# EUROPEAN PATENT APPLICATION

(11) **EP 0 554 624 A1**
(43) Date of publication of application: **11.08.1993**
(21) Application number: 92311780.8
(22) Date of filing: 24.12.1992
(51) Int. Cl.: C07K 15/00, C12N 15/51, G01N 33/576

(54) **Oligonucleotides, oligopeptides and determination system of HCV antibody subtype**

(30) Priority: 27.12.1991 JP 361488/91
(71) Applicant: IMMUNO JAPAN INC., Tokyo 167 (JP)
(72) Inventor: Okamoto, Hiroaki, Shimotsuga-gun Tochigi-ken 329-05, (JP); Nakamura, Tetsuo, Ogikubo Suginami-ku Tokyo 167 (JP)
(74) Representative: Watkins, Arnold Jack

(57) **Abstract**

A method is disclosed to detect and to determine subtypes of HCV antibodies in a large number of samples efficiently, and to provide oligonucleotides and oligopeptides used in the method directly and indirectly. Also disclosed is a detection system and a determination system of HCV antibody using one or more of oligopeptides described in SEQ ID 3 and 4. Oligopeptides can be obtained by means of chemical synthesis. Also, oligopeptides can be obtained by means of gene technology with genetic recombination of oligonucleotides having the nucleotide sequence described in SEQ ID 1 and 2, and those coding for oligopeptides having the amino acid sequence described in SEQ ID 3 and 4, into a host cell.

## Description

### Background of the Invention

The present invention concerns oligonucleotides, oligopeptides, and detection system of HCV antibody (hereinafter "HCV antibody") and determination system of anti-hepatitis C virus antibody subtype using said peptides as antigen.

Viral hepatitis of which DNA and RNA of the causative viruses have been elucidated, and their diagnosis and even prevention in some cases have been established, are hepatitis A and hepatitis B. The general name NANB hepatitis was given to the other forms of viral hepatitis.

Post-transfusion hepatitis was remarkably reduced after introduction of diagnostic systems for screening hepatitis B virus in transfusion bloods. However, there are still an estimated 280,000 annual cases of post-transfusion hepatitis caused by NANB hepatitis virus in Japan.

NANB hepatitis viruses were recently named C, D and E according to their types, and scientists started a world wide effort to conduct research for the causative viruses.

In 1988, Chiron Corp. claimed that they had succeeded in cloning RNA virus genome, which they termed hepatitis C virus as the causative agent of NANB hepatitis and reported on its nucleotide sequence (British Patent 2,212,511 which is the equivalent of European Patent Application 0,318,216). HCV (C100-3) antibody detection systems based on the sequence are now being introduced for screening of transfusion bloods and for diagnosis of patients in Japan and in many other countries.

The detection systems for the C100-3 antibody have proven their partial association with NANB hepatitis; however, they capture only about 70% of carriers and chronic hepatitis patients, or they fail to detect the antibody in acute phase infection, thus leaving problems yet to be solved even after development of the C100-3 antibody by Chiron Corp.

HCV, or NANB hepatitis having a relationship to C100-3 antibody, has single stranded RNA which suggests some relationship to Flaviviruses and Pestiviruses. From the comparison of those genome structures, regions coding for core protein (C), envelope protein (E) and non-structure protein (NS) were found in HCV. C100-3 antibody detected by Chiron's ELISA kit is thought to recognize a part of the border region of NS3/NS4.

On the other hand, the course of NANB hepatitis is troublesome and many cases of horizontal transmission are considered to become carriers and then to develop chronic hepatitis. In addition, most patients with chronic hepatitis develop liver cirrhosis, then hepatocellular carcinoma. It is therefore very imperative to isolate the virus itself and to develop effective diagnostic reagents enabling earlier diagnosis.

The presence of a number of NANB hepatitis which can not be diagnosed by Chiron's aforementioned HCV (C100-3) antibody detection kits suggests the possible importance of detection of antibody to core protein instead of antibody to non-structural protein. From that view point, the genome of the core region and the protein coded for by this region have been studied world wide.

From the two-by-two comparison of nucleotide sequences of various HCV isolates, homologies for the core region, which is rather conservative, were found to be less than 90% in some cases. In addition, more variability was found in the envelope region (which is thought to be important for development of a vaccine); less than 60% homology was found between some isolates. The sequence diversity between strains reflects the features of HCV as RNA virus.

A rapid and simple method of classifying HCV genomes into some groups, therefore, would be very useful for diagnosis, therapy and prevention of NANB hepatitis.

Determination of HCV subtypes could be useful in the following medical and social applications:
(1) identification of infectious sources in vertical (mother to baby) transmission and horizontal transmission;
(2) clarification of subtype-specific features in the course, condition and prediction of recuperation of NANB liver diseases and preparing a guide for therapy therefore;
(3) increasing the detection sensitivity of HCV by nucleic acid detection system or antibody detection system based on the study of diversity in nucleotide or amino acid sequences among HCV isolates of distinct subtypes;
(4) production of type-specific immunoglobulin (IgG) and vaccine based on the study of type-specific conservative regions in envelope gene of various subtypes; and
(5) rapid and effective prevention by selecting an appropriate IgG or vaccine for each subtype.

The inventors have obtain various HCV-RNAs from sera of humans and chimpanzees, and cloned cDNAs of HCV genomes in non-coding regions and coding regions for core and envelope proteins, followed by determination of nucleotide sequences (Japanese Patent Application Nos. 196175/91, 287402/91 and 360441/91), and sought to find out a method to determine HCV genotypes and oligonucleotide primers to be used in said method, which would be useful in diagnosis, therapy, prevention, identification of infectious source, and epidemiological study of HCV. As a result, variation of nucleotide sequence characteristics in some groups in the core region, which was thought to be rather conservative, was found, and much more variation was found in the envelope region.

The inventors studied the genomes of a great number of HCV isolates, classified them by the nucleotide sequence of the core region, and found that HCV strains could be divided into groups. Further study on the subtypes of HCV led to the classification of HCV into four subtypes based on the homology of nucleotides; the inventors then completed a convenient and efficient determination system of HCv subtypes by polymerase chain reaction (hereinafter "PCR") using type-specific primers and amplification of specific regions of each subtype (Japanese Patent Application No. 307296/91, 93960/92, and 276502/92). Hereinafter, subtype determined by PcR is called genotype. HCV genomes were classified into four genotypes as type I through IV using said determination system.

As a result of analysis of amino-acid sequences of HCV isolates found by the inventors and HCV-1 found by Chiron Corp., the homology between types I and II was calculated as approximately 96% in the C region and approximately 79% in the E region, and the homology between types III and IV was calculated as approximately 95% in the C region and approximately 71% in the E region. On the other hand, between types I and IV and types II and III, the homology was calculated as low as approximately 91% in the C region of both cases and approximately 54% and 53% respectively in the E region. As a result, four genotypes were found to be divided into two groups, and that types I/II and types III/IV are respectively close.

However, genotyping of HCV by comparing nucleotide sequences of individual isolates, or more simply by PCR with genotype-specific primers, is not applicable if the RNA in serum or plasma samples has been lost through storage or laboratory handling, or if viremia has been cleared and only antibodies remain as markers of infection. Furthermore, such methods are time-consuming and are not convenient for routine use. The inventors have now established a method to detect subtype specific antibody serologically in order to determine large numbers of HCV subtypes more conveniently.

### Summary of the Invention

The present invention provides a method to detect subtypes of HCV antibodies easily, rapidly and definitely. Polypeptides of the invention are to be applied in the determination of subtypes of HCV antibodies.

The method of the invention improves sensitivity of detection system of HCV antibody since it provides a method to detect subtype-specific antibodies definitely. The invention provides many other applications, for example, development of IgG with high specificity for HCV subtype, development of vaccine with high specificity for HCV subtype, and establishment of ways to administer said IgG and vaccine.

Oligopeptides of the invention can be used as antigens in detection system and determination system of the invention.

Oligonucleotides of the invention provide oligopeptides of the invention, for example when genetically recombinated into proper host cell (e.g., *E. coli*).

More specifically, the present invention partially relates to the following:

Recombinant polynucleotide CN Type-1 of HCV, comprising the cDNA sequence in sequence list 1.

Recombinant polynucleotide CN Type-2 of HCV, comprising the cDNA sequence in sequence list 2.

Recombinant peptide CP Type-1 of HCV, comprising the peptide sequence in sequence list 3.

Recombinant peptide CP Type-2 of HCV, comprising the peptide sequence in sequence list 4.

A HCV antibody detection reagent comprising the peptide sequence according to sequence list 3 or 4 as antigen and a conventional vehicle.

A HCV diagnostic test kit for analyzing samples for the presence of antibodies directed against a HCV antigen, comprising the peptide sequence according to sequence list 3 or 4 as antigen attached to a solid substrate and labeled anti-human immunoglobulin.

A method for detecting subtype specific HCV antibodies in a sample involving:
(a) reacting the sample with the peptide according to sequence list 3 or 4 attached to a solid substrate under conditions which allow the formation of an antigen-antibody complex;
(b) reacting the product of step (a) with labelled anti-human IgG or IgM antibodies; and
(c) detecting the presence of labelled anti-human IgG or IgM antibodies bound to the antigen-antibody complex.

### Detailed Description of the Invention

HCV-RNAs were isolated from sera and plasma samples obtained from humans and chimpanzees and the nucleotide sequence of their cDNAs were determined. The amino acid sequence coded for by the HCV-RNAs were then determined. After that, the HCV genomes were classified into four genotypes using the determination system disclosed in japanese Patent Application No. 307296/91, 93960/92, and 276502/92.

Finally, the method of the invention to detect and determine HCV antibodies using type-specific peptides as antigen specified from the results of comparison of amino acid sequence between the two groups, types I/II and types III/IV, was completed.

Seven of the seventeen amino acids between isoleucine (65th position) through tyrosine (81st position) in the C region are different between groups I/II and III/IV. Based on this finding, CP-TYPE-1 of SEQ ID3 (Ile-Pro-Lys-Ala-Arg-Arg-Pro-Glu-Gly -Arg-Thr-Trp-Ala-Gln-Pro-Gly-Tyr) and CP-TYPE-2 of SEQ ID4 (Ile-Pro-Lys-Asp-Arg-Arg-Ser-Thr-Gly-Lys-Ser-Trp-Gly-Lys-Pro-Gly-Tyr) were synthesized by known methods discussed below. Underlined amino acids are those which differ between CP-TYPE-1 and 2 as antigen.

Two ELISA systems (TYPE-1, TYPE-2) were made to detect HCV antibody using CP-TYPE-1 and CP-TYPE-2 as antigen. Detection of HCV antibodies by the two ELISA systems was carried out over serum and plasma samples described above. The determination of subtypes by the ELISAs gave the same results as that by PCR on all of the nineteen samples which had high enough antibody titers (light absorbance degree of 0.3 or more) and had the proportion between light absorbances measured by TYPE-1 and TYPE-2 of 4 or more. As a result, the system of the invention was found to give determination of subtypes of antibodies with high fidelity.

In the method of the invention, the subtype having high titer of HCV antibody, which proportion of absorbances TYPE-1/TYPE-2 gives ≧ 4 is called serotype 1, while that with TYPE-2/TYPE-1 ≧ 4 is called serotype 2. Serotype I includes genotypes I and II and serotype 2 includes genotypes III and IV.

The present invention includes the following subjects:
oligonucleotide CN-TYPE-1 having nucleotide sequence comprising SEQ ID1;
oligonucleotide CN-TYPE-2 having nucleotide sequence comprising SEQ ID2;
oligopeptide CP-TYPE-1 having amino acid sequence comprising SEQ ID3;
oligopeptide CP-TYPE-2 having amino acid sequence comprising SEQ ID4;
oligopeptides (SEQ ID3, SEQ ID4) obtained by chemical synthesis;
oligopeptides (SEQ ID3, SEQ ID4) obtained by recombinant oligonucleotides (SEQ ID1, SEQ ID2) coding for oligopeptides;
detection system of HCV antibody using one or more of the above-described oligopeptides as antigen; and
determination system of subtypes of HCV antibody using one or more of the above-identified oligopeptides according as antigen.

Oligonucleotide CN-TYPE-1 is the nucleotide sequence which genotypes I and II generally have in the core region, but which genotypes III and IV do not have.

Oligonucleotide CN-TYPE-2 is the nucleotide sequence which genotypes III and IV generally have in the core region, but which genotypes I and II do not have.

The above-described oligopeptides can be obtained by genetic recombination using oligonucleotides (CN-TYPE-1 and CN-TYPE-2) into a host cell, e.g. *E. coli*. Besides that, oligonucleotides (CN-TYPE-1 and CN-TYPE-2) coding for oligopeptides give said oligopeptides.

Classification of HCV genomes obtained from Japanese sera shows that type II is up to 82% of the sera, III is 10%, and I and IV are 4% in asymptomatic carriers or normal group.

However, type II was found to be present in 60% and type III in 23% of patients with liver diseases, where the population of genotypes was different between normal group (Japanese Patent Application 307296/91, 93960/92, and 276502/92).

The method of the invention can be utilized in therapy, prevention and prospect of clinical course for patients with type C liver disease since the possibility of developing liver disease is significantly different among HCV subtypes. In addition, the method of the invention provides confirmation of HCV double infection and the infectious source of horizontal and vertical transmission of HCV.

The method of the invention differs from the determination system for genotypes in some points, for example:
(1) it is more technologically convenient;
(2) it needs a shorter time to treat many samples; and
(3) it is possible to determine the subtype of HCV from "memory" antibody in the serum which does not contain RNA or the virus itself.

Examples of applications of these inventions are shown below, however, the invention shall in no way be limited to those examples.

### Examples

### Experimental Examples-Method to determine genotype by PCR

### 1. Isolation of RNA

RNA of human serum or plasma samples which tested positive for HCV antibody (C100-3 antibody by Ortho HCV Ab ELISA, ortho Diagnostic Systems, Tokyo) were isolated by the following method:

Serum or plasma sample was added with Tris chloride buffer (10mM, pH 8.0) and centrifuged at 68 x 10³ rpm for an hour. The precipitate was suspended in Tris chloride buffer (50mM, pH 8.0) containing 200mM NaCl, 10mM EDTA, 2% (W/V) sodium dodecyl sulfate (SDS), and proteinase K 1mg/ml, and was incubated at 60°C for one hour. Then its nucleic acid was extracted by phenol/chloroform and precipitated by ethanol to obtain RNA.

### 2. Amplification by Polymerase Chain Reaction (PCR)

After heating the isolated RNA at 70°C for 1 minute, this was used as a template and cDNA was synthesized using oligonucleotide primer #186 (5'-GCCGACCTCATGGGGTACAT-3', nt 391-410, antisense) and reverse transcriptase Superscript (BRL, U.S.A.) and was provided to genotype PCR.

PCR was carried out by the following reaction cycle: denaturation at 94°C for 1 minute, annealing of primers at 60°C for 1 minute, and extension at 72°C for 1.5 minutes. First stage PCR and second stage PCR were carried out for 30 cycles.

The products were subjected to electrophoresis on a composite agarose gel made from 1.5% NuSieve and 1.5% SeaKem (FMC BioProducts, U.S.A.), stained with ethidium bromide, and observed under u.v. light.

Nucleotide of 271 bases, nt 139-410, was amplified in first stage PCR using universal primers #256 (5'-CGCGCGACTAGGAAGACTTC-3', nt 139-158, sense) and #186, then 1/50 amount of the product was subjected to the second stage PCR. In the second stage PCR, universal primer #104 (5'-AGGAAGACTTCCGAGCGGTC-3', nt 148-167, sense) was used as sense primer, and equivalent mixture of type-specific primers #132 (5'-GTCAGCCTATCCCCAAGGCA-3', nt 185-204, antisense), #133 (5'-TGGGGTGGGCAGGATGGCTC-3', nt 272-291, antisense), #134 (5'-GAGGTTCCCGTCCCTCTTGG-3', nt 302-321, antisense), #135 (5'-CTCTGTACGGAAACGAGGGT-3', nt 251-270, antisense) as antisense primer. PCR products were observed after electrophoresis, and each band was confirmed by the primer among #132 - #135 that it was amplified by. Subtypes were named types I, II, III and IV for products amplified by #132, #133, #134 and #135 respectively.

### Example 1-Manufacture of peptides

Using the well-known method described by Merrifield, peptides CP-TYPE-1 (having the amino acid sequence Ile-Pro-Lys-Ala-Arg-Art-Pro-Glu-Gly-Arg-Thr-Trp-Ala-Gln-Pro-Gly-Tyr) and CP-TYPE-2 (having the amino acid sequence Ile-Pro-Lys-Asp-Arg-Arg-Ser-Thr-Gly-Lys-Ser-Trp-Gly-Lys-Pro-Gly-Tyr) were synthesized by condensation of amino acid of C-terminus of the peptide with polystyrene followed by condensation with t-butoxy carbonyl amino acids one by one directed to N-terminus.

Then the amino acid compositions were confirmed by hydrolysis with 6N HCl for 24 hours at 110°C under reduced pressure.

### Example 2-Detection system for HCV antibody

The detection system was developed using polyvinyl microtiter plates and the sandwich EIA method.

CP-TYPE-1 and CP-TYPE-2 obtained by chemical synthesis in Example 1 were used as solid phase antigen. 50µl of 10mM phosphate buffer (pH 7.5) containing 5µg/ml concentration of the peptide was dispensed in each well of the microtiter plate (96 wells, Greiner, Germany) for ELISA and incubated overnight at room temperature. The wells were washed with physiological saline containing 0.05% Tween 20, then 100µl of NaCl buffer containing 40% (V/V) of calf serum was dispensed in each well and discarded after incubation for 30 minutes at room temperature.

For determination of samples, in the primary reaction 50µl of physiological saline containing 40% calf serum and 0.05% Tween 20 and 10µl of a sample was dispensed in each microplate well and incubated on a microplate vibrator for one hour at room temperature. After completion of the reaction, microplate wells were washed five times in the same way as previously described.

In the secondary reaction, as labeled antibody 1ng of horseradish peroxidase labeled anti-human IgG mouse monoclonal antibodies dissolved in 50µl of calf serum was dispensed in each microplate well and incubated on a microplate vibrator for one hour at room temperature. Wells were washed five times in the same way as previously described . After addition of sodium perborate (as substrate) and 50µl of O-phenylendiamine solution (as color developer) in each well, and after incubation for 30 minutes at room temperature, 50µl of 4N sulfuric acid was dispensed in each well to stop further color development and for reading absorbance at 492nm.

Antibodies with various labels can be utilized in the secondary reaction (see U.S. Patent 4,873,188 which is incorporated by reference).

In this example, the criteria for determining the subtype of HCV antibody were defined as follows:
(1) if either light absorbance degree of CP-TYPE-1 (hereinafter Abs.1) and light absorbance degree of CP-TYPE-2 (hereinafter Abs.2) is equal to or more than 0.3, and if the proportion of Abs.1/Abs.2 is equal to or more than 4.0, the subtype was determined as serotype 1; and
(2) if either Abs.1 and Abs.2 is equal to or more than 0.3, and if the proportion of Abs.1/Abs.2 is equal to or less than 0.25, the subtype was determined as serotype 2.

Since the criteria were employed only for this example, the criteria per se do not constitute an essential element of this invention.

### Example 3

To confirm that the determination system of HCV antibody of the invention is useful in epidemiological research, serum samples obtained from inhabitants of areas (A to T) where the frequency of patients with liver diseases was found to be very high were subjected to the determination test. The results are shown in Table 1; subtypes were determined for 94 samples, 56 (60%) were found to be serotype 1 and 38 (40%) were found to be serotype 2.

Seventeen samples were selected at random from the 94 samples in which serotypes were determined, and subjected to determination system of genotypes as described in the Experimental Example after isolating RNA. The results are shown in Table 2; the genotype completely harmonized with the serotype respectively in all of seventeen samples.

As described above, since the determination system of HCV antibody of the invention harmonizes with results of genotype determination system using PCR, and since large numbers of samples can be treated to determine their subtypes easily using the present system, it was confirmed that the system of the invention was very useful.

**Table 1**

| AREA | CASE | SEROTYPE | |
|---|---|---|---|
| | | 1 | 2 |
| A | 2 | 2 | 0 |
| B | 4 | 3 | 1 |
| C | 8 | 6 | 2 |
| D | 1 | 0 | 1 |
| E | 2 | 2 | 0 |
| F | 3 | 0 | 3 |
| G | 1 | 0 | 1 |
| H | 1 | 1 | 0 |
| I | 5 | 3 | 2 |
| J | 9 | 3 | 6 |
| K | 8 | 4 | 4 |
| L | 4 | 4 | 0 |
| M | 6 | 3 | 3 |
| N | 3 | 3 | 0 |
| O | 5 | 3 | 2 |
| P | 1 | 1 | 0 |
| Q | 2 | 1 | 1 |
| R | 4 | 2 | 2 |
| S | 9 | 6 | 3 |
| T | 16 | 9 | 7 |
| TOTAL | 94 | 56 | 38 |

**Table 2**

| Sample No. | EIA (Absorbance at 492 mm) | | SEROTYPE | GENOTYPE |
|---|---|---|---|---|
| | TYPE-1 | TYPE-2 | | |
| 1 | 2.248 | 0.160 | 1 | II |
| 2 | 0.042 | 0.856 | 2 | III |
| 3 | 0.205 | 2.083 | 2 | III |
| 4 | 2.220 | 0.036 | 1 | II |
| 5 | 0.852 | 0.140 | 1 | II |
| 6 | 0.973 | 0.074 | 1 | II |
| 7 | 0.027 | 0.472 | 2 | IV |
| 8 | 0.051 | 2.364 | 2 | III |
| 9 | 0.113 | 0.744 | 2 | III |
| 10 | 2.300 | 0.282 | 1 | II |
| 11 | 2.227 | 0.034 | 1 | II |
| 12 | 0.064 | 1.750 | 2 | III |
| 13 | 0.951 | 0.037 | 1 | II |
| 14 | 2.258 | 0.035 | 1 | II |
| 15 | 2.203 | 0.028 | 1 | II |
| 16 | 2.176 | 0.109 | 1 | II |
| 17 | 0.090 | 1.488 | 2 | III |

The detection system and the determination system of the present invention provides a convenient, speedy, and sure way to determine the subtype of HCV antibody of large numbers of samples. The method of the invention differs on those points from the determination system of HCV genotype using PCR.

The method of the invention can be useful in therapy, prevention and prospect of clinical course for patient with type C liver disease, confirmation of double infection and infectious source of horizontal and vertical transmission. Moreover, it provides many applications, e.g., development of subtype-specific IgG and vaccine, and establishment of method to administer them.

Oligonucleotides and oligopeptides of the invention are used in the detection system and determination system of the invention directly or indirectly.

It will also be understood that the practice of the present invention is not limited to the use of the exact sequence of CN Type-1 and CN Type-2. Modifications to the sequence, such as deletions, insertions, or substitutions in the sequence which produce silent changes in the resulting protein molecule are also contemplated. For example, alteration in the gene sequence which reflect the degeneracy of the genetic code, or which result in the production of a chemically equivalent amino acid at a given site, are contemplated; thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, are also expected to produce a biologically equivalent product. Nucleotide changes which result in alteration of the N-terminal and C-terminal portions of the protein molecule would also not be expected to alter the activity of the protein. Therefore, where the phrase CN Type-1 or CN Type-2 is used in either the specification or the claims, it will be understood to encompass all such modifications and variations which result in the production of a biologically equivalent product. In particular, the invention contemplates those nucleic acid sequences which are sufficiently duplicative of CN Type-1 or CN Type-2 so as to permit hybridization therewith under standard high stringency southern hybridization conditions, such as those described in Maniatis et al. (Molecular Cloning. A Laboratory Manual, 2nd ed. Cold Spring Harbor Laboratory, 1989), or encode proteins which react with antisera to CP Type-1 or CP Type-2.

It is well known in the art that one or more amino acids in an amino acid sequence can be replaced by equivalent other amino acids, as demonstrated by U.S. Patent No. 4,737,487 which is incorporated by reference, in order to produce an analog of the amino acid sequence. Any analogs of CP Type-1 or CP Type-2 of the present invention involving amino acid deletions, amino acid replacements, such as replacements by other amino acids, or by isosteres (modified amino acids that bear close structural and spatial similarity to protein amino acids), amino acid additions, or isosteres additions can be utilized, so long as the sequences elicit antibodies recognizing antigens CP Type-1 or CP Type-2.

Polypeptides can be utilized in a HCV related antibody detection reagent which contains such polypeptides and a conventional vehicle.

Polypeptides of the invention are useful as material to produce polyclonal and monoclonal antibodies by known techniques. Polyclonal and monoclonal antibodies of the invention are useful as materials for diagnostic agents to detect HCV antigens with high specificity. Antigen-antibody complexes can be detected by methods known in this art. Specific monoclonal and polyclonal antibodies can be obtained by immunizing such animals as mice, guinea pigs, rabbits, goats and horses with polypeptides (e.g., HCV antigenic epitope(s)).

Specific antibodies directed against HCV can be utilized in a HCV related antigen detection reagent which contains such specific antibodies and a conventional vehicle.

Further variations and modifications of the foregoing will be apparent to those skilled in the art and such variations and modifications are attended to be encompassed by the claims that are appended hereto.

Japanese Priority Application 361488/91 filed on December 27, 1991 is relied on and incorporated by reference. Machida, A., et al., "Two Distinct Subtypes Of Hepatitis C Virus Defined By Antibodies Directed To The Putative Core Protein", Hepatology (1992), volume 16, pages 886-891, is incorporated by reference in its entirety.

Japanese Patent Application No. 196175/91 and U.S. Patent Application Serial No. 07/866,045 (filed on April 9, 1992); Japanese Patent Application Nos. 287402/91 and 360441/91, and U.S. Patent Application Serial No. 07/925,695 (filed on August 7, 1992); and Japanese Patent Application Nos. 307296/91, 93960/92, and 276502/92), and U.S. Patent Application Serial No. 07/940,242 (filed on September 8, 1992) are incorporated by reference.

The terms "functionally equivalent" and "degenerate" sequences when used in connection with polynucleotides include sequences which encode peptides having HCV antigencity but are modified by single or multiple nucleotide substitutions, additions and/or deletions. The term "antigenically significant fragment" includes fragments of the peptides disclosed herein which possess an antigenic activity.

Sequence List

SEQ ID LIST 1 (CN-TYPE-1)
(A) LENGTH: 51 base pairs
(B) TYPE: nucleic acid
(C) STRANDEDNESS: single
(D) TOPOLOGY: linear
(E) MOLECULE TYPE:cDNA to genomic RNA

SEQUENCE DESCRIPTION OF SEQ ID LIST 1

ATCCCCAAGG TGCGTCGGCC CGAGGGCAGG ACCTGGGCTC
AGCCCGGGTA C 51

SEQ ID LIST 2 (CN-TYPE-2)
(A) LENGTH: 51 base pairs
(B) TYPE: nucleic acid
(C) STRANDNESS: single
(D) TOPOLOGY: linear
(E) MOLECULE TYPE:cDNA to genomic RNA

ATCCCCAAGG ATCGGCGCTC CACTGGCAAA TCCTGGGGAA
AACCAGGATA C 51

SEQUENCE ID LIST 3 (CP-TYPE-1)
'(A) LENGTH: 17 amino acids
(B) TYPE: amino acid
(D) TOPOLOGY: linear
(E) MOLECULE TYPE:peptide

SEQUENCE DESCRIPTION OF SEQ ID LIST 3
Ile Pro Lys Ala Arg Arg Pro Glu Gly Arg
                5                   10
Thr Trp Ala Gln Pro Gly Tyr 17
                15

SEQ ID LIST 4 (CP-TYPE-2)
(A) LENGTH: 17 amino acids
(B) TYPE: amino acid
(D) TOPOLOGY: linear
(E) MOLECULE TYPE:peptide

SEQUENCE DESCRIPTION OF SEQ ID LIST 4'

Ile Pro Lys Asp Arg Arg Ser Thr Gly Lys
                5                   10
Ser Trp Gly Lys Pro Gly Tyr 17
                15

## Claims

1. A polynucleotide of HCV, comprising the cDNA sequence of sequence list 1 or sequence list 2, and functionally equivalent or degenerate variants thereof.

2. A polynucleotide as claimed in claim 1 being a recombinant molecule.

3. A synthetic peptide of HCV, comprising the peptide sequence of sequence list or sequence list 4 and antigenically significant fragments thereof.

4. A peptide as claimed in claim 3 being a recombinant peptide.

5. A DNA molecule encoding the peptide sequence of claim 3.

6. An HCV antibody detection reagent comprising a peptide as claimed in claim 3 as antigen.

7. An HCV diagnostic test kit for analyzing samples for the presence of antibodies directed against an HCV antigen, comprising a) a peptide according to claim 3 as antigen; and b) labelled anti-human immunoglobulin.

8. A kit as claimed in claim 7 wherein the peptide is attached to a solid substrate.

9. A method for detecting subtype specific HCV antibodies in a sample comprising:
(a) reacting said sample with a peptide according to claim 3 under conditions which allow the formation of an antigen-antibody complex;
(b) reacting the product of step (a) with labelled anti-human IgG or IgM antibodies; and
(c) detecting the presence of labelled anti-human IgG or IgM antibodies bound to said antigen-antibody complex.

10. A method as claimed in claim 9 wherein in step (a) the peptide is attached to a solid substrate.
